# EUROPÄISCHE PATENTANMELDUNG

(11) **EP 3 949 917 A1**
(43) Veröffentlichungstag der Anmeldung: **09.02.2022**
(21) Anmeldenummer: 20189292.4
(22) Anmeldetag: 04.08.2020
(51) Int. Cl.: A61F 5/44, A61F 5/453

(54) **OBERKÖRPER-TRAGESYSTEM ZUR URINSAMMLUNG UND URINABLEITUNG BEI INKONTINENZPATIENTEN**

(71) Anmelder: Zimmermann, Heinz, 82340 Feldafing (DE)
(72) Erfinder: Zimmermann, Heinz, 82340 Feldafing (DE)
(74) Vertreter: Kaufmann, Tobias

(57) **Zusammenfassung**

Die Erfindung betrifft ein System und ein Verfahren zur Urinsammlung und Urinableitung bei einem/einer Inkontinenzpatienten/-in. Das System umfasst einen elastisch ausgebildeten Hüllenabschnitt, wobei der elastisch ausgebildete Hüllenabschnitt in einen Schlauchabschnitt übergeht und einen Pufferbereich umfasst, einen Sammelbehälter, der einen Adapter mit Rücklaufventil und mindestens ein Verschlussmittel umfasst, wobei der Sammelbehälter zur Anbringung am Torso vorgesehen ist, und wobei der Schlauchabschnitt mit dem Adapter mit Rücklaufventil des Sammelbehälters verbunden ist.

## Beschreibung

### GEBIET DER ERFINDUNG

Die vorliegende Erfindung betrifft ein System und ein Verfahren zur Urinsammlung und Urinableitung bei Inkontinenzpatienten.

### HINTERGRUND DER ERFINDUNG

Harninkontinenz, oder auch Blasenschwäche, bezeichnet jeglichen unfreiwilligen Verlust von Urin.

Zur Abhilfe werden Patienten bereits ableitende Inkontinenzsysteme bestehend aus Urinal-Kondom, Verbindungsschlauch und Urinbeutel in unterschiedlichen Größen und Bauarten angeboten. Bei den bisher bekannten Systemen ist der Urinbeutel üblicherweise unterhalb des Urinal-Kondoms am Bein eines Anwenders angebracht. Das Tragen des Urinbeutels am Bein kann für den Anwender jedoch von Nachteil sein. Insbesondere ist eine Befestigung am Bein beim Sport hinderlich und der Urinbeutel kann nach außen hin sichtbar sein. Darüber hinaus wird es dem Anwender erschwert, kurze Hosen zu tragen, ohne dass der Urinbeutel sichtbar wird.

Eine weitere Behandlungsmöglichkeit für Patienten bietet der Einsatz eines Blasenkatheters, wobei ein Kunststoffschlauch entweder über die Harnröhre oder die Bauchdecke in die Harnblase eingebracht wird. Die Verwendung von Blasenkathetern ermöglicht das Tragen von Urinbeuteln in Form von Hüftbeuteln. Die Verwendung von Blasenkathetern führt jedoch zu einem erhöhten gesundheitlichen Risiko und erhöht insbesondere die Infektionsgefahr. Ebenso bedarf die Verwendung von Blasenkathetern ärztlicher Anleitung und Einweisung und wird von den Anwendern oft als umständlich wahrgenommen.

Somit liegt der vorliegenden Erfindung die Aufgabe zugrunde, ein Oberkörper-Tragesystem zur Urinsammlung und Urinableitung bei Inkontinenzpatienten zu entwickeln, welches gesundheitliche Risiken minimiert, ohne dabei den Tragekomfort zu mindern.

Diese Aufgabe wird durch ein System und ein Verfahren gemäß der vorliegenden Erfindung gelöst.

### ZUSAMMENFASSUNG DER ERFINDUNG

Die vorliegende Erfindung betrifft ein System zur Urinsammlung und Urinableitung bei einem/einer Inkontinenzpatienten/-in. Das System umfasst einen elastisch ausgebildeten Hüllenabschnitt, wobei der elastisch ausgebildete Hüllenabschnitt in einen Schlauchabschnitt übergeht und einen Pufferbereich umfasst. Zusätzlich umfasst das System einen Sammelbehälter, der einen Adapter mit Rücklaufventil und mindestens ein Verschlussmittel umfasst, wobei der Sammelbehälter zur Anbringung am Torso vorgesehen ist, wobei der Schlauchabschnitt mit dem Adapter mit Rücklaufventil des Sammelbehälters verbunden ist.

Der Sammelbehälter kann ein Urinbeutel sein. Urinbeutel können in unterschiedlichen Größen und Bauarten angeboten werden, damit sie den verschiedenen Ansprüchen des Anwenders gerecht werden. Beispielsweise kann das Fassungsvermögen von Urinbeuteln zwischen 500 ml und 3000 ml variieren.

Die Bereitstellung eines Pufferbereichs führt dazu, dass eine eintretende Menge Urin aufgenommen werden kann und anschließend in den Sammelbehälter weitergeleitet werden kann.

Der Adapter mit Rücklaufventil verhindert dabei, dass von dem Sammelbehälter bereits aufgenommener Urin, nicht mehr zurück in den Pufferbereich gelangen kann.

Das mindestens eine Verschlussmittel des Sammelbehälters ermöglicht dem Verwender des Systems eine vereinfachte Entleerung des Sammelbehälters.

Die Anbringung des Sammelbehälters am Torso, bietet im Vergleich zur Anbringung am Bein, ästhetische Vorteile und darüber hinaus eine verbesserte Beweglichkeit, beispielsweise beim Sport.

In einer Ausführungsform kann der Schlauchabschnitt direkt mit dem Adapter am Sammelbehälter verbunden werden. Ein zusätzlicher Verbindungsschlauch ist dabei nicht notwendig.

In einer anderen Ausführungsform kann der Schlauchabschnitt über einen Verbindungsschlauch mit dem Adapter des Rücklaufventils verbunden werden.

In einer weiteren Ausführungsform, die für die Verwendungen von männlichen Patienten vorgesehen ist, umfasst das System ein Urinal-Kondom, das den elastisch ausgebildeten Hüllenabschnitt und den Schlauchabschnitt umfasst.

Ein Urinal-Kondom (auch Kondomurinal) stellt ein gängiges ableitendes Inkontinenzsystem-Hilfsmittel dar. Im Allgemeinen sind Urinal-Kondome meist aus Gummi, Latex oder Silikon gefertigt und besitzen die Form eines Kondoms mit Anschlussmöglichkeit eines Schlauches. Im Vergleich zu der Verwendung eines invasiven Blasenkatheters sind Urinal-Kondome einfacher zu handhaben und bieten ein geringeres gesundheitliches Risiko. In besonders vorteilhafter Weise ist dabei zur Befestigung des Urinal-Kondoms ein umschlingendes Band, das hinter den Hoden befestigt wird, vorgesehen. Das umschlingende Band kann beispielsweise um das Urinal-Kondom befestigt werden, oder, weiter bevorzugt, mit einer Festhalteklemme an das Urinal-Kondom angebracht werden. Dies verhindert in vorteilhafter Weise ein Abrutschen des Urinal-Kondoms über die Eichel und damit den unerwünschten Urin-Austritt.

Bei einer weiteren Ausführungsform wird der Pufferbereich gebildet, indem der elastisch ausgebildete Hüllenabschnitt an einem Penisschaft befestigt wird.

In einer weiteren Ausführungsform kann das System ein Befestigungsmittel zur Befestigung des elastisch ausgebildeten Hüllenabschnitts an einem Penisschaft umfassen.

Dies ermöglichst eine auslaufsichere Anwendung des Systems für den Anwender. Insbesondere ermöglicht die Verwendung eines Befestigungsmittels, eine Ansammlung von Urin im Pufferbereich ohne Urinverlust.

In einer weiteren Ausführungsform kann das Befestigungsmittel ein Klebemittel umfassen. Das Klebemittel kann beispielsweise ein Klebeband und/oder einen Klebstoff umfassen.

In einer weiteren Ausführungsform kann das Befestigungsmittel ein mechanisches Kompressionsmittel, umfassen. Das mechanische Kompressionsmittel kann hierbei eine Feststellklemme und/oder ein Gummilitzenband mit Feststellklemme umfassen. Durch ein stufenlos verstellbares Gummilitzenband mit Feststellklemme und wählbarer Gummielastizität kann eine optimale Anpassung an unterschiedliche anatomische Verhältnisse ermöglicht werden.

Nach der erfindungsgemäßen Lehre kann der Pufferbereich, der von einem Teil des elastisch ausgebildeten Hüllenabschnittes des Urinal-Kondoms gebildet wird, eine eintretende Urinmenge aufnehmen. Hierdurch wird der elastisch ausgebildete Hüllenabschnitt des Pufferbereichs gedehnt. Dies führt zu einem geänderten Druck im dem elastisch ausgebildeten Hüllenabschnitt des Pufferbereichs, und somit zu einer Saugwirkung z.B. durch ein Teilvakuum in dem geschlossenen System, wodurch Urin zu den Sammelbehälter weitertransportiert werden kann. Dies ermöglicht somit, dass der Sammelbehälter an einem gegenüber den Genitalbereich des Anwenders erhöhten Niveau befestigt werden kann und der austretende Urin entgegen der Schwerkraft in den höher gelegenen Sammelbehälter geführt werden kann.

Dieses Prinzip lässt sich auch bei Patientinnen oder Patienten mit anomalen Penis anwenden: Bei dieser Ausführungsform kann der Pufferbereich gebildet werden, indem der elastische Hüllenabschnitt durch einen elastischen Schlauch- bzw. Katheter-Bereich, eine sog. elastische Katheterblase, zwischen Sammelbehälter und Blase des Anwenders bereitgestellt wird. Vorzugsweise ist diese Katheterblase zum Anschluss an einen invasiv eingeführten Kathether vorgesehen.

Die Verwendung einer Katheterblase ermöglicht so beispielsweise die Anwendung des Systems in Verbindung mit einem invasiven Blasenkatheter. Insbesondere wird dadurch auch Frauen die Verwendung des beanspruchten Systems ermöglicht.

Bei einer weiteren Ausführungsform überschreitet der Pufferabschnitt und der Schlauchabschnitt zusammen eine Länge von 10 cm, vorzugsweise von 7 cm, weiter vorzugsweise von 5 cm nicht.

Dies ermöglicht, dass der in den Pufferbereich eingetretene Urin in den Sammelbehälter weiterbefördert werden kann.

In einer weiteren Ausführungsform kann der Adapter mit Rücklaufventil an einem unteren Ende des Sammelbehälters angeordnet sein.

Dies ermöglich eine Anbringung des Sammelbehälters am Torso, wobei die Länge von Pufferabschnitt und Schlauchabschnitt zusammen möglichst klein gehalten werden kann.

In einer weiteren Ausführungsform kann das mindestens eine Verschlussmittel an einem oberen Ende und/oder einer Seite und/oder einem unteren Ende des Sammelbehälters angeordnet sein.

In einer weiteren Ausführungsform kann der Sammelbehälter im Brustbereich, und/oder Bauchbereich, und/oder Hüftbereich angeordnet werden.

In einer anderen Ausführungsform kann der Sammelbehälter seitlich am Torso angeordnet sein.

In einer anderen Ausführungsform kann das System gegenüber der Umgebung luftdicht geschlossen sein. Damit wird verhindert, dass eintretende Luft die Ausbildung der oben beschriebenen Druckverhältnisse verhindert, so dass der Transport des austretenden Urins entgegen der Schwerkraft in einen ggf. höher gelegenen Sammelbehälter ermöglicht wird.

In einer weiteren Ausführungsform kann der Sammelbehälter mit einem verstellbaren Tragemittel am Torso angeordnet werden.

Dadurch kann die Anordnung des Sammelbehälters individuell für den Anwender angepasst werden.

In einer weiteren Ausführungsform kann das Tragemittel zum Tragen um einen Nacken ausgebildet sein, wenn der Sammelbehälter zur Anordnung im Brustbereich als Bauchbeutel vorgesehen ist.

In einer weiteren Ausführungsform kann das Tragemittel zum Tragen um einen Bauch ausgebildet sein.

Die vorliegende Erfindung betrifft auch ein Verfahren zur Urinsammlung und Urinableitung bei einem/einer Inkontinenzpatienten/-in. Das Verfahren kann Aufnehmen von Urin durch einen Pufferbereich umfassen, wobei ein elastisch ausgebildeter Hüllenabschnitt, der in einen Schlauchabschnitt übergeht, den Pufferbereich umfassen kann. Weiterhin kann das Verfahren Weiterleiten des Urins von dem Pufferbreich zu einen Sammelbehälter umfassen, der einen Adapter mit Rücklaufventil und mindestens ein Verschlussmittel umfasst, wobei der Sammelbehälter zur Anbringung am Torso vorgesehen sein kann, wobei der Schlauchabschnitt mit dem Adapter des Rücklaufventils verbunden sein kann.

### KURZE BESCHREIBUNG DER ZEICHNUNGEN

Die Erfindung, sowie ihre neuartigen Merkmale und ihre Ausgestaltung, werden aus den anhängigen Zeichnungen im Zusammenhang mit der Beschreibung veranschaulicht.
Fig. 1 zeigt ein bekanntes System, zur Urinsammlung und Urinableitung bei einem Inkontinenzpatienten.
Fig. 2 zeigt ein System, zur Urinsammlung und Urinableitung bei einem Inkontinenzpatienten gemäß einer Ausführungsform der vorliegenden Erfindung.
Fig. 3 zeigt ein System, zur Urinsammlung und Urinableitung bei einem Inkontinenzpatienten gemäß einer anderen Ausführungsform der vorliegenden Erfindung.
Fig. 4 zeigt ein erfindungsgemäßes Urinal-Kondom mit einem leeren Pufferbereich.
Fig. 5 zeigt ein erfindungsgemäßes Urinal-Kondom mit einem mit Urin gefüllten Pufferbereich.
Fig. 6 zeigt ein Ablaufdiagramm, welches ein Verfahren zur Urinsammlung und Urinableitung bei einem/einer Inkontinenzpatienten/-in gemäß Ausführungsformen der vorliegenden Erfindung veranschaulicht.
Fig. 7 zeigt ein System zur Urinsammlung und Urinableitung bei einem Inkontinenzpatienten gemäß einer weiteren Ausführungsform der vorliegenden Erfindung.
Fig. 8 zeigt ein System zur Urinsammlung und Urinableitung bei einer weiblichen Inkontinenzpatientin gemäß einer weiteren Ausführungsform der vorliegenden Erfindung.

### DETAILLIERTE BESCHREIBUNG DER ZEICHNUNGEN

Nachfolgend werden Ausführungsbeispiele der Erfindung anhand von Zeichnungen veranschaulicht.

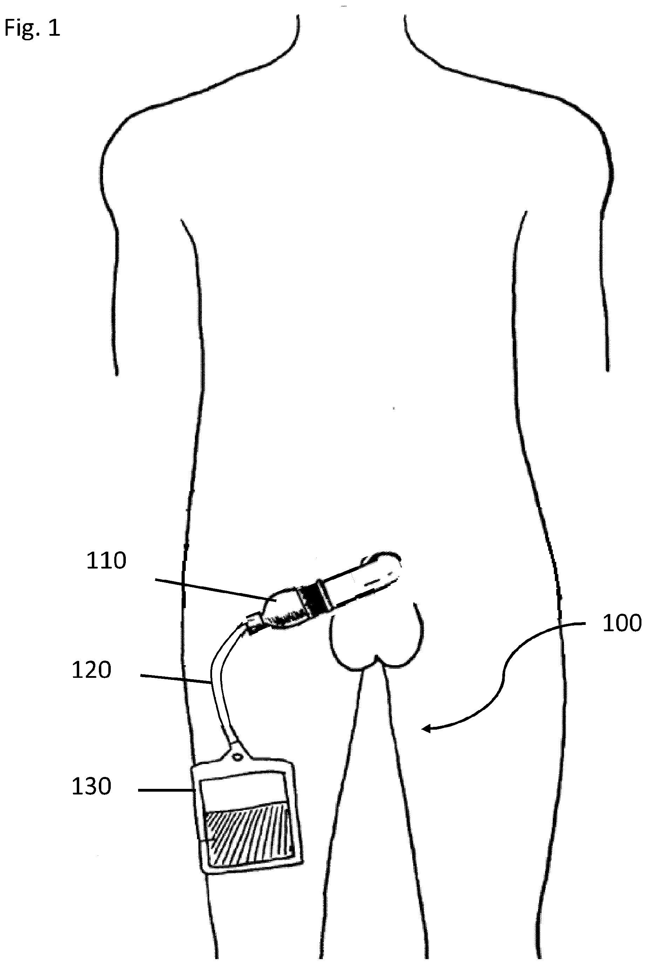

Fig. 1 zeigt eine vereinfachte Darstellung eines gängigen Systems 100, zur Urinsammlung und Urinableitung bei Inkontinenzpatienten. Das dargestellte System 100 umfasst ein Urinal-Kondom 110 mit Anschlussmöglichkeit eines Verbindungsschlauches 120, der zu einem an einem Bein befestigten Urinbeutel 130 führt. Die Verwendung eines Systems mit Urinal-Kondom 110 bietet gegenüber der Versorgung mit Blasenkathetern den Vorteil einer einfacheren Handhabung und eines geringeren gesundheitlichen Risikos. Dabei wird der Urinbeuel 130 am Bein des Anwenders, unterhalb des Urinal-Kondoms 110 befestigt. Hierbei wird Urin mithilfe der Schwerkraft in Richtung Urinbeutel weitergeleitet. Ein Tragen des Urinbeutels am Bein, kann für den Anwender von Nachteil sein. Insbesondere kann eine Befestigung am Bein beim Sport hinderlich sein und der Urinbeutel kann nach außen hin sichtbar sein.

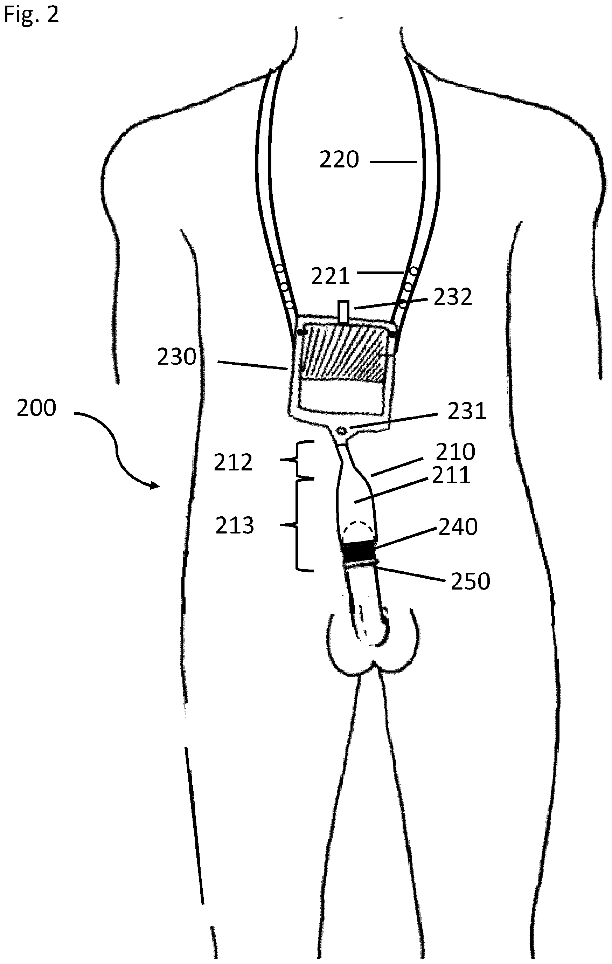

Fig. 2 zeigt ein System 200, zur Urinsammlung und Urinableitung bei Inkontinenzpatienten gemäß einer Ausführungsform der vorliegenden Erfindung. Das System umfasst einen elastisch ausgebildeten Hüllenabschnitt 213 wobei der elastisch ausgebildete Hüllenabschnitt 213 in einen Schlauchabschnitt 212 übergeht und einen Pufferbereich 211 umfasst bzw. ausbildet. Zusätzlich umfasst das System 200 einen Sammelbehälter 230, der einen Adapter mit Rücklaufventil 231 und mindestens ein Verschlussmittel 232 umfassen kann, wobei der Sammelbehälter zur Anbringung am Torso vorgesehen ist. Der Schlauchabschnitt 212 kann mit dem Sammelbehälter 230 und insbesondere mit dem Adapter mit Rücklaufventil 231 verbunden sein.

In Fig. 2 umfasst das System ein Urinal-Kondom 210, das den elastisch ausgebildeten Hüllenabschnitt 213 und den Schlauchabschnitt 212 umfasst. Dies stellt insbesondere eine Ausführungsform des erfindungsgemäßen Systems dar, wie es für den Einsatz bei männlichen Patienten vorgesehen ist. Bei weiblichen Patientinnen, oder bei männlichen Patienten mit anomalem Penis, ist die Verwendung eines Urinal-Kondoms nicht möglich. Hier ist anstelle des Urinalkondoms wie in Fig. 2 gezeigt eine elastische Katheterblase, vorzugsweise aus Gummi, vorgesehen (nicht gezeigt in Fig. 2), welche die Pumpfunktion des Pufferbereichs 211 übernimmt. Diese elastische Katheterblase wird zwischen Sammelbehälter und Blase des Anwenders bereitgestellt und ist vorzugsweise zum Anschluss an einen invasiv eingeführten Kathether, z.B. einen Dauerkatheter, vorgesehen. Die Katheterblase wird dabei vorzugsweise durch eine Haltevorrichtung am Körper fixiert, so dass auch bei dieser Ausführungsform der beabsichtigte Tragekomfort erreicht wird.

Der Sammelbehälter 230 kann ein Urinbeutel sein. Urinbeutel können in unterschiedlichen Größen und Bauarten angeboten werden, damit sie den verschiedenen Ansprüchen des Anwenders gerecht werden. Beispielsweise kann das Fassungsvermögen von Urinbeuteln zwischen 500 ml und 3000 ml variieren.

Ein Urinal-Kondom (auch Kondomurinal) stellt ein gängiges ableitendes Inkontinenzsystem-Hilfsmittel dar. Im Allgemeinen sind Urinal-Kondome meist aus Gummi, Latex oder Silikon gefertigt und besitzen die Form eines Kondoms mit Anschlussmöglichkeit eines Schlauches. Im Vergleich zu der Verwendung eines Blasenkatheters sind Urinal-Kondome einfacher zu Handhaben und bieten ein geringeres gesundheitliches Risiko. In besonders vorteilhafter Weise ist dabei zur Befestigung des Urinal-Kondoms ein umschlingendes Band (in der Figur nicht gezeigt), das hinter den Hoden befestigt wird, vorgesehen. Das umschlingende Band kann beispielsweise um das Urinal-Kondom befestigt werden, oder, weiter bevorzugt, mit einer Festhalteklemme an das Urinal-Kondom angebracht werden. Dies verhindert in vorteilhafter Weise ein Abrutschen des Urinal-Kondoms über die Eichel und damit den unerwünschten Urin-Austritt.

Zur Anbringung des Urinal-Kondoms am Penis bietet sich die Verwendung einer Manschette als Abrollhilfe an. Diese sollte dabei einen etwas größeren Durchmesser als der Penis aufweisen.

Der Pufferbereich 211 ist dazu geeignet eine eintretende Menge Urin aufzunehmen und anschließend in den Sammelbehälter 230 weiterzuleiten.

Der Adapter mit Rücklaufventil 231 verhindert dabei, dass Urin, der bereits von dem Sammelbehälter 230 aufgenommen wurde, wieder zurück in das Urinal-Kondom 211, bzw. in den Pufferbereich gelangen kann.

In der dargestellten Ausführungsform kann der Schlauchabschnitt direkt an dem Adapter am Sammelbehälter befestigt werden. Ein zusätzlicher Verbindungsschlauch ist dabei nicht notwendig.

In einer anderen Ausführungsform kann der Schlauchabschnitt über einen Verbindungsschlauch mit dem Adapter des Rücklaufventils verbunden werden.

Wie in Figur 2 vereinfacht dargestellt, kann bei dem System der offenbarten Erfindung, der Sammelbehälter am Torso befestigt werden. Dies bietet im Vergleich zur Befestigung am Bein ästhetische Vorteile und darüber hinaus eine verbesserte Beweglichkeit für den Anwender.

In einer weiteren Ausführungsform wird der Pufferbereich 211 gebildet, indem der elastisch ausgebildete Hüllenabschnitt 213 an einem Penisschaft 250 befestigt wird.

Der Pufferbereich 211, der von einem Teil des elastisch ausgebildeten Hüllenabschnittes 213 des Urinal-Kondoms 210 gebildet wird, kann eine eintretende Menge an Urin aufnehmen. Hierdurch kann der elastisch ausgebildete Hüllenabschnitt 213 im Pufferbereich 211 gedehnt werden. Dies führt zu einem geänderten Druck in dem vorliegenden geschlossenen System wodurch Urin zu dem Sammelbehälter 230 weitertransportiert wird.

Bei einer weiteren Ausführungsform überschreitet der Pufferabschnitt 211 eine Länge von 10 cm, vorzugsweise von 7 cm, weiter vorzugsweise von 5 cm nicht.

Dies ermöglicht, dass der in den Pufferbereich eingetretene Urin, in den Sammelbehälter weiterbefördert werden kann.

In einer weiteren Ausführungsform kann der Adapter mit Rücklaufventil 231 an einem unteren Ende des Sammelbehälters 230 angeordnet sein. Dies ermöglich eine Anbringung des Sammelbehälters 230 am Torso, wobei die Länge von dem Pufferabschnitt 211 möglichst klein gehalten werden kann.

In einer weiteren Ausführungsform kann das mindestens eine Verschlussmittel 232 an einem oberen Ende und/oder einer Seite und/oder einem unteren Ende des Sammelbehälters angeordnet sein.

In der in Figur 2 dargestellten Ausführungsform kann der Sammelbehälter 230 im Brustbereich, und/oder Bauchbereich, z.B. im Brustbereich als Bauchbeutel, angeordnet werden.

In einer anderen Ausführungsform kann der Sammelbehälter 230 aber auch im Hüftbereich und/oder seitlich am Torso angeordnet werden.

In einer weiteren Ausführungsform kann der Sammelbehälter mit einem verstellbaren Tragemittel 220 am Torso angeordnet werden. Dadurch kann die Anbringung des Sammelbehälters individuell für den Anwender angepasst werden.

Figur 2 zeigt eine mögliche Ausführungsform, bei der das Tragemittel 220 zum Tragen um einen Nacken ausgebildet ist, wenn der Sammelbehälter 230 zur Anordnung im Bauchbereich vorgesehen ist. Hierbei kann das Tragemittel 220 beispielsweise mit Hilfe von Knöpfen, Druckknöpfen 221 (wie in Figur 2 bespielhaft gezeigt), Trageschleife, oder sonstigen anpassbaren Trägern variabel auf den Anwender eingestellt werden.

In einer anderen, nicht in Figur 2 gezeigten, Ausführungsform kann das Tragemittel zum Tragen um einen Bauch ausgebildet sein. Das Tragemittel kann hierbei einen verstellbaren Bauchgurt umfassen.

In der in Figur 2 dargestellten Ausführungsform, umfasst das System weiterhin ein Befestigungsmittel 240 zur Befestigung des elastisch ausgebildeten Hüllenabschnitts 213 an einem Penisschaft 250.

Dies ermöglichst eine auslaufsichere Verwendung des Systems 200 für den Anwender. Insbesondere ermöglicht die Verwendung eines Befestigungsmittels 240, eine Ansammlung von Urin im Pufferbereich 211 ohne Urinverlust.

In einer weiteren Ausführungsform kann das Befestigungsmittel 240 ein Klebemittel umfassen.

In einer weiteren Ausführungsform kann das Befestigungsmittel 240 ein mechanisches Kompressionsmittel umfassen. Das mechanische Kompressionsmittel kann hierbei eine Feststellklemme und/oder ein Gummilitzenband mit Feststellklemme umfassen. Durch ein stufenlos verstellbares Gummilitzenband mit Feststellklemme und wählbarer Gummielastizität kann eine optimale Anpassung an unterschiedliche anatomische Verhältnisse ermöglicht werden.

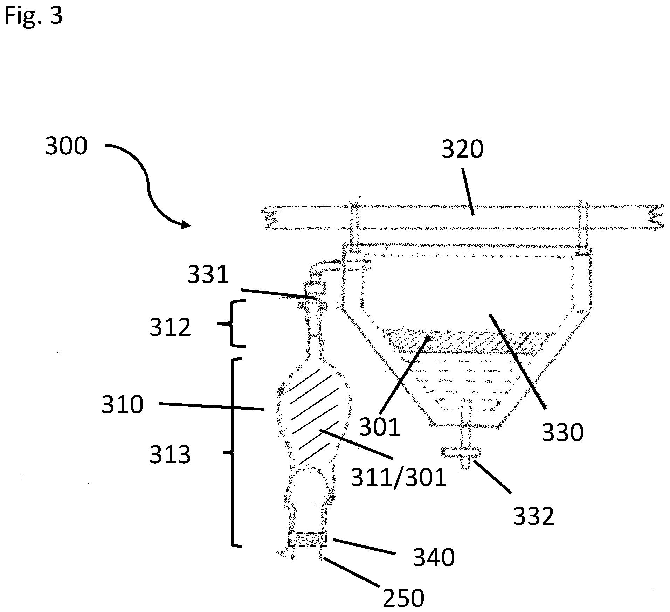

Fig. 3 zeigt ein System 300, zur Urinsammlung und Urinableitung bei Inkontinenzpatienten gemäß einer anderen Ausführungsform der vorliegenden Erfindung. Das System umfasst einen elastisch ausgebildeten Hüllenabschnitt 313, der in einen Schlauchabschnitt 312 übergeht, wobei der elastisch ausgebildete Hüllenabschnitt 313 einen Pufferbereich 311 umfasst. Zusätzlich umfasst das System einen Sammelbehälter 330, der einen Adapter mit Rücklaufventil 331 und mindestens ein Verschlussmittel 332 umfassen kann, wobei der Sammelbehälter 330 zur Anbringung am Torso vorgesehen ist, wobei der Schlauchabschnitt 312 mit dem Adapter mit Rücklaufventil 331 verbunden werden kann.

In Fig. 3 umfasst das System ein Urinal-Kondom 310, das den elastisch ausgebildeten Hüllenabschnitt 313 und den Schlauchabschnitt 312 umfasst.

Wie in Bezug auf Figur 2 erläutert, kann der Pufferbereich 311 gebildet werden, indem der elastisch ausgebildete Hüllenabschnitt 313 an einem Penisschaft 250 befestigt wird.

Der Pufferbereich 311, der von einem Teil des elastisch ausgebildeten Hüllenabschnittes 313 des Urinal-Kondoms 310 gebildet wird, kann eine eintretende Menge an Urin 301 aufnehmen. Hierdurch kann der elastisch ausgebildete Hüllenabschnitt 313 im Pufferbereich 311 gedehnt werden (wie in Figur 3 vereinfacht dargestellt). Dies kann zu einem geänderten Druck in dem vorliegenden System führen, wodurch Urin 301 in den Sammelbehälter 330 weitertransportiert werden kann.

In der in Figur 3 gezeigten Ausführungsform kann das mindestens eine Verschlussmittel 332 an einem unteren Ende des Sammelbehälters 330 angeordnet werden.

In der in Figur 3 dargestellten Ausführungsform kann der Sammelbehälter 330 seitlich am Torso, am Bauch oder Hüfte angeordnet werden.

In einer weiteren Ausführungsform kann der Sammelbehälter mit einem verstellbaren Tragemittel 320 am Torso angeordnet werden. Hierzu bietet sich beispielsweise eine um den Hals getragene Vorrichtung an, z.B. ein Band, an welches der Sammelbehälter mit Hilfe von Druckknöpfen befestigt wird. Insbesondere können die Druckknöpfe dabei so ausgestaltet sein, dass der Sammelbehälter auf unterschiedlichen Höhen getragen werden kann. In einer alternativen Ausgestaltung wird das verstellbare Tragemittel 320 durch Klettverschlüsse gebildet. In der in Figur 3 dargestellten Ausführungsform kann das Tragemittel 320 auch zum Tragen um einen Bauch ausgebildet sein. Das Tragemittel kann hierbei einen verstellbaren Bauchgurt umfassen.

An den Sammelbehälter kann weiterhin ein Ablaufschlauch, beispielsweise mit Schiebe- oder Drehverschluss, zur Entleerung des Sammelbehälters angebracht sein. Die Länge des Ablaufschlauches würde vorzugsweise bis zu 15 cm betragen.

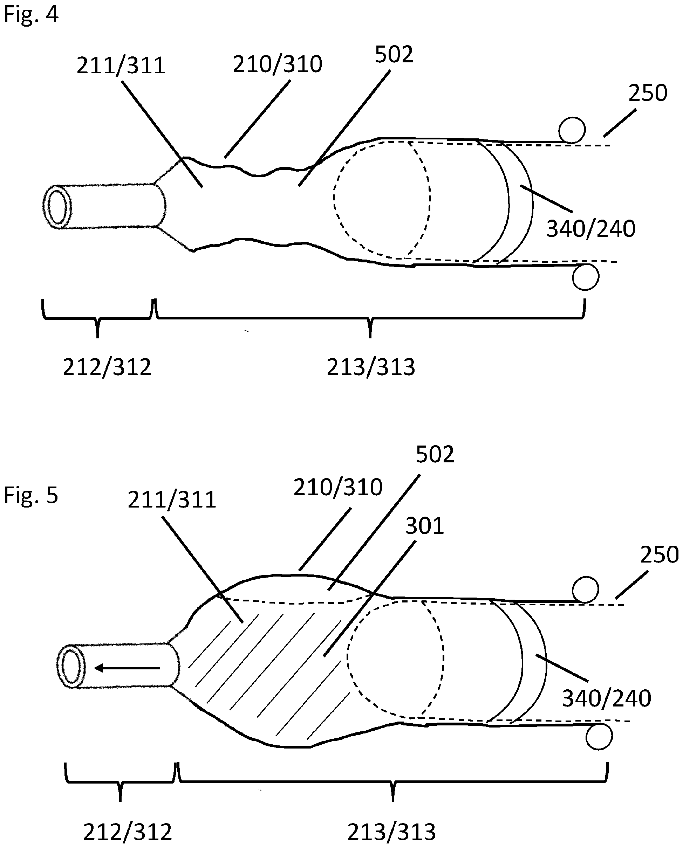

Fig. 4 zeigt ein erfindungsgemäßes Urinal-Kondom 210/310 mit einem leeren Pufferbereich 211/311. In der in Figur 4 gezeigten Ausführungsform kann der Pufferbereich 211/311 gebildet werden, indem der elastisch ausgebildete Hüllenabschnitt 213/313 an einem Penisschaft 250 befestigt wird. Hierzu kann der elastisch ausgebildete Hüllenabschnitt 213/313 mit Hilfe eines Befestigungsmittels 240/340 an einem Penisschaft 250 befestigt werden. Wie in Figur 4 vereinfacht dargestellt, weist der leere Pufferbereich 211/311 lediglich eine geringe Menge an Luft 502 auf.

Fig. 5 zeigt ein erfindungsgemäßes Urinal-Kondom 210/310, das einen mit Urin 301 gefüllten Pufferbereich 211/311 aufweist. Wie in Fig. 5 vereinfacht dargestellt kann der Pufferbereich 211/311 des Urinal-Kondoms eine eintretende Menge an Urin 301 aufnehmen. Durch die umfasste Luftmenge 502 und die eingetretene Urinmenge 301 vergrößert sich das Volumen des elastisch ausgebildeten Hüllenabschnitts 213/313 im Pufferbereich 211. Dies kann zu einem gedehnten Hüllenabschnitt 213/313 im Pufferbereich 211 führen und kann, da von außen weder Luft eintritt noch welche abgeführt wird, wiederum zu einem geänderten Druck in Form eines Unterdrucks in dem gedehnten Hüllenabschnitt 213/313 in dem Pufferbereich führen. Durch die geänderten Druckverhältnisse in dem geschlossenen System, die sich auch auf den mit dem Schlauchabschnitt verbundenen Sammelbehälter 230/320, welcher ohne Verwendung eines Verbindungsschlauchs an dem Urinal-Kondom befestigt werden kann, erstrecken, kann Urin zu dem Sammelbehälter 230/320 weitertransportiert werden. Während der Erfinder auf die Wirkweise der Erfindung aufgrund der tatsächlichen Notwendigkeit nach einer Alternative zu vorbekannten Systemen gestoßen ist, lässt sich dieses Prinzip auch mit Hilfe eines Wasserhahns - anstelle des Penis - und der beschriebenen Ausgestaltung nachvollziehen. Hierfür kann das Urinal-Kondom mit einer Feststellklemme an einen üblichen Wasserhahn angeschlossen werden. Das Wirkprinzip lässt sich so anschaulich nachvollziehen.

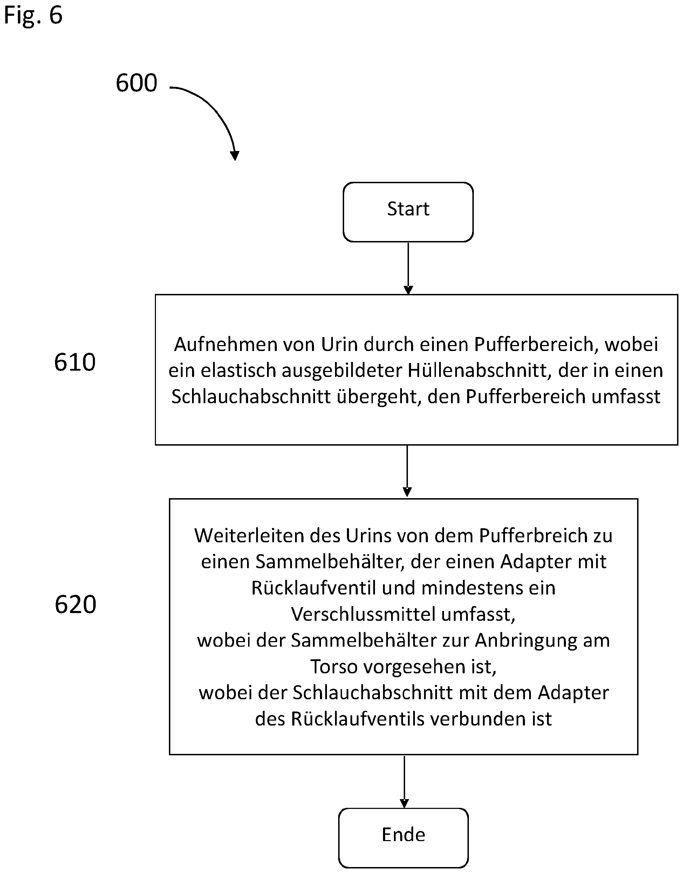

Fig. 6 zeigt ein Ablaufdiagramm 600, welches ein Verfahren zur Urinsammlung und Urinableitung bei einem/einer Inkontinenzpatient/-in gemäß Ausführungsformen der vorliegenden Erfindung veranschaulicht. Das Verfahren umfasst Aufnehmen 610 von Urin durch einen Pufferbereich, wobei ein elastisch ausgebildeter Hüllenabschnitt, der in einen Schlauchabschnitt übergeht, den Pufferbereich umfasst.

Aufnehmen 610 von Urin durch den Pufferbereich kann zu einem vergrößerten Volumen des elastisch ausgebildeten Hüllenabschnitts im Pufferbereich führen. Dies kann zu einem gedehnten Hüllenabschnitt im Pufferbereich führen und kann wiederum zu einem geänderten Druck in dem gedehnten Hüllenabschnitt im Pufferbereich führen. Durch die geänderten Druckverhältnisse im System kann Urin zu dem Sammelbehälter 230/320 weitertransportiert werden.

Weiterhin umfasst das Verfahren Weiterleiten 620 des Urins von dem Pufferbreich zu einem Sammelbehälter, der einen Adapter mit Rücklaufventil und mindestens ein Verschlussmittel umfassen kann, wobei der Sammelbehälter zur Anbringung am Torso vorgesehen sein kann, wobei der Schlauchabschnitt mit dem Adapter des Rücklaufventils verbunden sein kann.

In einer weiteren Ausführungsform wird der elastisch ausgebildete Hüllenabschnitt und der Schlauchabschnitt von einem Urinal-Kondom bereitgestellt.

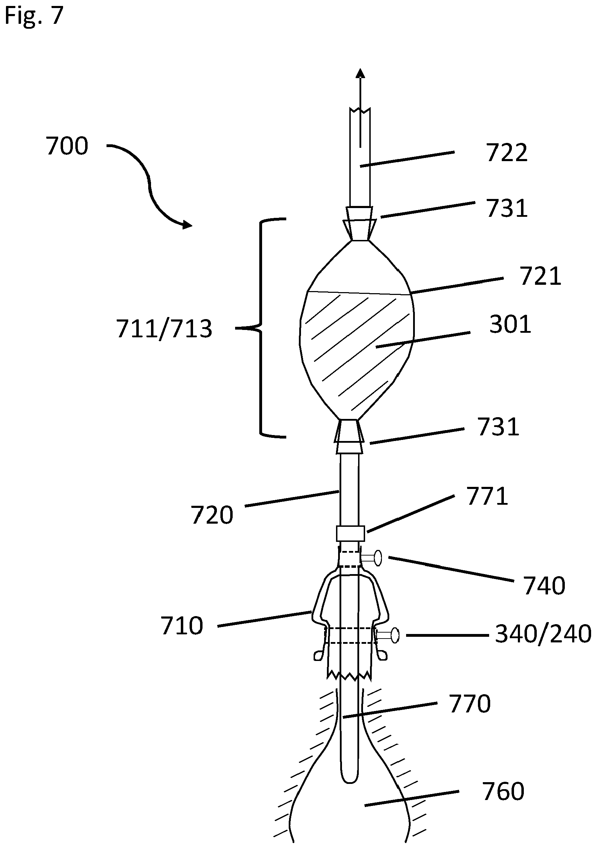

Fig. 7 zeigt ein System 700 zur Urinsammlung und Urinableitung bei einem Inkontinenzpatienten gemäß einer weiteren Ausführungsform der vorliegenden Erfindung. Fig. 7 zeigt die Anwendung des erfindungsgemäßen Systems in Verbindung mit einem Blasenkatheter 770. Bei der in Fig. 7 dargestellten Ausführungsform kann der Pufferbereich 711 gebildet werden, indem der elastische Hüllenabschnitt 713 durch einen elastischen Schlauch- bzw. Katheter-Bereich 721, eine sog. elastische Katheterblase, zwischen Sammelbehälter und Harnblase 760 des Anwenders, bereitgestellt wird. In Fig. 7 ist die Katheterblase 721 über einen Katheteranschluss 771 an einen invasiv eingeführten Blasenkathether 770 angeschlossen. Somit lässt sich das System 700 auch bei Patienten mit anomalen Penis anwenden.

In der in Fig. 7 dargestellten Ausführungsform kann der Blasenkatheter 770 über Befestigungsmittel 740, vorzugsweise eine oder mehrere Feststellklemmen, an einem Urinal-Kondom 710 zusätzlich befestigst werden.

In der in Fig. 7 dargestellten Ausführungsform kann der elastische Schlauch- bzw. Katheter-Bereich 721 über einen Verbindungschlauch 720 an den Blasenkatheter 770 angeschlossen werden. Weiterhin kann der elastische Schlauch- bzw. Katheter-Bereich 721 über einen weiteren Verbindungsschlauch 722 mit einem Sammelbehälter (nicht in Fig. 7 gezeigt) verbunden werden. Dies entspricht insofern der bereits im Hinblick auf Fig. 2 und 3 beschriebenen Vorgehensweise.
In einer anderen Ausführungsform können die in Fig. 7 dargestellten Verbindungsschläuche 720/722 und der elastische Schlauch- bzw. Katheter-Bereich 721 auch von einem einzigen Schlauch bzw. Katheter mit unterschiedlichen Schlauch-Elastizitätsbereichen bereitgestellt werden. Dies ermöglicht es, den elastischen Schlauch- bzw. Katheter-Bereich 721 bereitzustellen ohne die Verwendung von Adaptern 731 und/oder eines Katheteranschlusses 771.

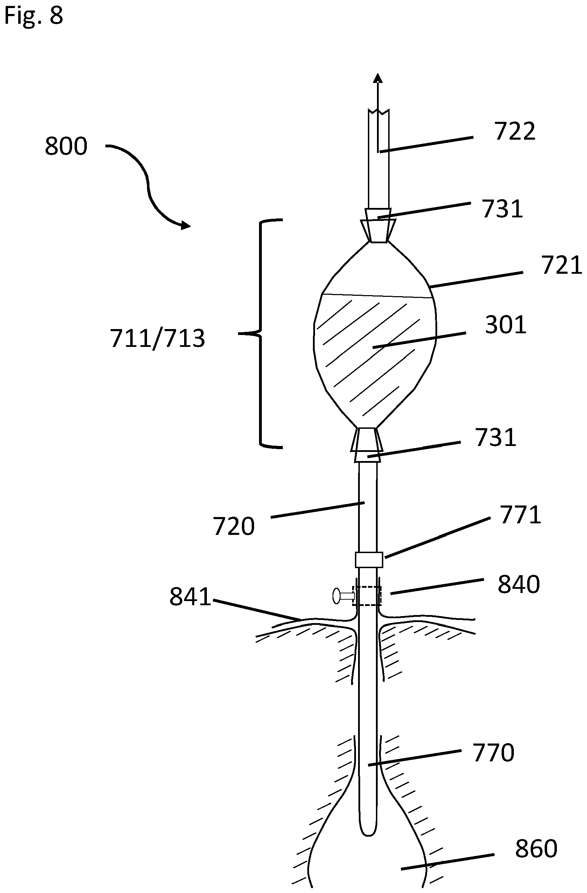

Fig. 8 zeigt ein System 800 zur Urinsammlung und Urinableitung bei einer weiblichen Inkontinenzpatientin gemäß einer weiteren Ausführungsform der vorliegenden Erfindung. Bei der in Fig. 7 dargestellten Ausführungsform kann der Pufferbereich 711 gebildet werden, indem der elastische Hüllenabschnitt 713 durch einen elastischen Schlauch- bzw. Katheter-Bereich 721, eine sog. elastische Katheterblase, zwischen Sammelbehälter (nicht in Fig. 8 gezeigt) und Harnblase 860 der Anwenderin, bereitgestellt wird. In Fig. 8 ist die Katheterblase 721 über einen Katheteranschluss 771 an einen invasiv eingeführten Blasenkatheter 770 angeschlossen.

Dies ermöglicht die Verwendung des erfindungsgemäßen Systems durch weibliche Patienten, da kein Urinal-Kondom verwendet werden muss, um den elastischen Hüllenabschnitt bereitzustellen.

In der in Fig. 8 dargestellten Ausführungsform kann der elastische Schlauch- bzw. Katheter-Bereich 721 über einen Verbindungschlauch 720 an den Blasenkatheter 770 angeschlossen werden. Weiterhin kann der elastische Schlauch- bzw. Katheter-Bereich 721 über einen weiteren Verbindungsschlauch 722 mit einem Sammelbehälter (nicht in Fig. 7 gezeigt) verbunden werden.

In einer anderen Ausführungsform können die Verbindungsschläuche 720/722 und der elastische Schlauch- bzw. Katheter-Bereich 721 von einem Schlauch bzw. Katheter mit unterschiedlichen Schlauch-Elastizitätsbereichen bereitgestellt werden. Dies ermöglicht es, den elastischen Schlauch- bzw. Katheter-Bereich 721 bereitzustellen ohne die Verwendung von Adaptern 731 und/oder eines Katheteranschlusses 771.

In der in Fig. 8 dargestellten Ausführungsform, kann der Blasenkatheter 770 wahlweise über ein oder mehrere Befestigungsmittel 840/841 zusätzlich befestigst werden. In der Fig. 8 gezeigten Ausführungsform wird eine Klebefolie 841 am Harnröhreneingang angebracht und über eine Feststellklemme 840 an dem Blasenkatheter 770 befestigt.

### LISTE DER BEZUGSZEICHEN

- 100, 200, 300, 600, 700:: System
- 110, 210, 310, 710:: Urinal-Kondom
- 120, 720, 722:: Verbindungsschlauch
- 130, 230, 330:: Urinbeutel/Sammelbehälter
- 211, 311, 711:: Pufferbereich
- 212, 312:: Schlauchabschnitt
- 213, 313, 713:: elastisch ausgebildeter Hüllenabschnitt
- 220,320:: Tragemittel
- 221:: Druckknöpfe/Knöpfe
- 231, 331:: Adapter mit Rücklaufventil
- 232,332:: Verschlussmittel
- 240, 340, 740, 840, 841:: Befestigungsmittel
- 250:: Penisschaft
- 301:: Urinmenge
- 502:: Luft
- 721:: elastischer Schlauch- bzw. Katheter-Bereich
- 731:: Adapter
- 760, 860:: Harnblase
- 770:: Blasenkatheter
- 771:: Katheteranschluss

## Patentansprüche

1. System zur Urinsammlung und Urinableitung bei einem/einer Inkontinenzpatienten/-in umfassend:
einen elastisch ausgebildeten Hüllenabschnitt, wobei der elastisch ausgebildete Hüllenabschnitt in einen Schlauchabschnitt übergeht und einen Pufferbereich umfasst;
einen Sammelbehälter, der einen Adapter mit Rücklaufventil und mindestens ein Verschlussmittel umfasst;
wobei der Sammelbehälter zur Anbringung am Torso vorgesehen ist, und
wobei der Schlauchabschnitt mit dem Adapter mit Rücklaufventil des Sammelbehälters verbunden ist.

2. System nach Anspruch 1, weiterhin umfassend ein Urinal-Kondom, das den elastisch ausgebildeten Hüllenabschnitt und den Schlauchabschnitt umfasst, wobei vorzugsweise zur Befestigung des Urinal-Kondoms ein umschlingendes Band, das hinter den Hoden befestigt wird, vorgesehen ist und insbesondere mit einer Festhalteklemme an das Urinal-Kondom angebracht wird.

3. System nach Anspruch 2, wobei der Pufferbereich gebildet wird, indem der elastisch ausgebildete Hüllenabschnitt an einem Penisschaft befestigt wird.

4. System nach Anspruch 3, wobei das System weiterhin ein Befestigungsmittel zur Befestigung des elastisch ausgebildeten Hüllenabschnitts an dem Penisschaft umfasst.

5. System nach Anspruch 4, wobei das Befestigungsmittel ein Klebemittel und/oder ein mechanisches Kompressionsmittel, vorzugsweise eine Feststellklemme umfasst.

6. System nach Anspruch 1, weiterhin umfassend eine elastische Katheterblase, die den elastisch ausgebildeten Hüllenabschnitt und den Schlauchabschnitt umfasst, und vorzugsweise zum Anschluss an einen invasiv eingeführten Katheter vorgesehen ist.

7. System nach einem der vorhergehenden Ansprüche, wobei der Pufferbereich eine Länge von 10 cm, vorzugsweise von 7 cm, weiter vorzugsweise von 5 cm nicht überschreitet.

8. System nach einem der vorhergehenden Ansprüche, wobei der Adapter mit Rücklaufventil an einem unteren Ende des Sammelbehälters angeordnet ist.

9. System nach einem der vorhergehenden Ansprüche, wobei das mindestens eine Verschlussmittel an einem oberen Ende und/oder einer Seite und/oder an einem unteren Ende des Sammelbehälters angeordnet ist.

10. System nach einem der vorhergehenden Ansprüche, wobei der Sammelbehälter im Brustbereich, und/oder Bauchbereich, und/oder Hüftbereich angeordnet ist.

11. System nach einem der vorhergehenden Ansprüche, wobei das System gegenüber der Umgebung luftdicht geschlossen ist.

12. System nach einem der vorhergehenden Ansprüche, wobei der Sammelbehälter mit einem verstellbaren Tragemittel am Torso angeordnet wird.

13. System nach Anspruch 12, wobei das Tragemittel zum Tragen um einen Nacken oder zum Tragen um einen Bauch ausgebildet ist, wenn der Sammelbehälter zur Anordnung im Bauchbereich vorgesehen ist.

14. System nach einem der vorhergehenden Ansprüche, wobei der Schlauchabschnitt über einen Verbindungsschlauch mit dem Adapter des Rücklaufventils des Sammelbehälters verbunden ist.

15. Verfahren zur Urinsammlung und Urinableitung bei einem/einer Inkontinenzpatienten/-in umfassend:
Aufnehmen von Urin durch einen Pufferbereich, wobei ein elastisch ausgebildeter Hüllenabschnitt, der in einen Schlauchabschnitt übergeht, den Pufferbereich umfasst;
Weiterleiten des Urins von dem Pufferbreich zu einem Sammelbehälter, der einen Adapter mit Rücklaufventil und mindestens ein Verschlussmittel umfasst;
wobei der Sammelbehälter zur Anbringung am Torso vorgesehen ist,
wobei der Schlauchabschnitt mit dem Adapter des Rücklaufventils verbunden ist.
